# EUROPEAN PATENT APPLICATION

(11) **EP 3 991 778 A1**
(43) Date of publication of application: **04.05.2022**
(21) Application number: 20831004.5
(22) Date of filing: 24.06.2020
(51) Int. Cl.: A61M 37/00

(54) **MICRONEEDLE ARRAY HAVING UNEVEN NEEDLE DENSITY**

(30) Priority: 25.06.2019 JP 2019117926; 03.12.2019 JP 2019219179
(71) Applicant: Cosmed Pharmaceutical Co., Ltd., Kyoto-shi, Kyoto 601-8014 (JP)
(72) Inventor: QUAN, Ying-shu, Kyoto-shi, Kyoto 601-8014 (JP); LI, Ying-zhe, Kyoto-shi, Kyoto 601-8014 (JP); YAMASHITA, Hirofumi, Kyoto-shi, Kyoto 601-8014 (JP); KAJIYAMA, Kenji, Kyoto-shi, Kyoto 601-8014 (JP); KAMIYAMA, Fumio, Kyoto-shi, Kyoto 601-8014 (JP)
(74) Representative: Ter Meer Steinmeister & Partner
(86) International application number: PCT/JP2020/024859
(87) International publication number: WO 2020/262473

(57) **Abstract**

The present invention solves a problem that it is difficult for a microneedle located in a central portion of a microneedle array to be inserted into a skin. A microneedle array including a substrate and a plurality of microneedles arranged vertically and horizontally on one face of the substrate, wherein a needle interval of the microneedles is different in a peripheral portion and in a central portion of the substrate, and a needle density of the microneedles is different in the peripheral portion and in the central portion of the substrate. It is preferable that the needle density of the microneedles is sparser in the central portion than in the peripheral portion of the substrate, and the needle interval of the microneedles is wider in the central portion than in the peripheral portion of the substrate.

## Description

### TECHNICAL FIELD

The present invention relates to a technique of a microneedle array having a non-uniform needle density.

### BACKGROUND ART

As a method for administering a drug into a human body, oral administration and transdermal administration are often used. Injection is a typical transdermal administration method. However, injection is a procedure which takes time and labor of specialist such as physicians and nurses and is painful, and therefore many people do not welcome the procedure. In contrast, a transdermal administration method without pain using a microneedle array has been recently attracting attention (Non-Patent Document 1).

In the transdermal administration of a drug, skin stratum corneum works as a barrier to drug permeation, and sufficient permeability is not always provided by only applying the drug on a skin surface. In contrast, perforation into stratum corneum by using a minute needle, that is, a microneedle, can remarkably improve drug permeation efficiency compared with the application method. An article in which a large number of microneedles are integrated on a substrate is a microneedle array. A product in which an adhesive sheet for adhering the microneedle array to a skin, a release sheet for protecting an adhesive face, and the like are added to the microneedle array to facilitate its use is called a microneedle patch.

As a material of the microneedle, metal or silicon has been initially used, but thereafter, various polymer materials have been attracting attention from the viewpoint of processability. In particular, when a microneedle is produced using a substance that disappears by metabolism in the body such as a carbohydrate as a material, an accident does not occur even if the needle breaks and remains in the skin.

In microneedle patches known in patent documents, academic literatures, and the like, a microneedle array substrate has a flat face, and fine needles having a uniform length stand vertically on the substrate at a uniform density. Microneedle patches which have been produced by the inventors of the present invention and have been filed for patent up to now are also included in the category (Patent Documents 1 and 2). It is described that the microneedles disclosed in Patent Documents 1 and 2 have a (circular) cone shape, a (circular) truncated cone shape, or a konide shape, and the pitch between the microneedles is preferably 0.4 to 1.0 mm. In addition, various improvements have been made regarding a microneedle drug product and a method for producing the same, which have been filed for patent in recent years, for the purpose of ensuring puncture into a skin with microneedles (Patent Documents 3 and 4), the purpose of making microneedles uniformly carry a drug (Patent Documents 5 and 6), and the like.

Patent Document 3 describes that a needle-shaped body device (microneedle array) is arranged such that a protrusion has a pyramid shape and a blade formed by two side faces of the pyramid shape is directed to a tangential line of a circle having a predetermined position on a substrate surface as a center point.

Patent Document 4 describes that in a dissolvable microneedle drug product containing hyaluronic acid and the like, the distance between adjacent needle parts is substantially equal, about 1 to 10 needles are arranged per 1 mm, and the density of the needle parts is preferably 100 to 10000 needles per 1 cm².

It is described that the microneedle array produced by the production method of a microneedle array of Patent Document 5 preferably has a needle density of 1 to 200 needles/cm² from the viewpoint of being able to administer a predetermined drug without pain.

It is described that the microneedle device produced by the production method of a microneedle device of Patent Document 6 has a row of needles provided at intervals to have a density of about 1 to 10 needles per 1 mm, separated from each other by an equal distance with respect to the space of the needles in the row, and has a needle density of 100 to 10000 needles per 1 cm².

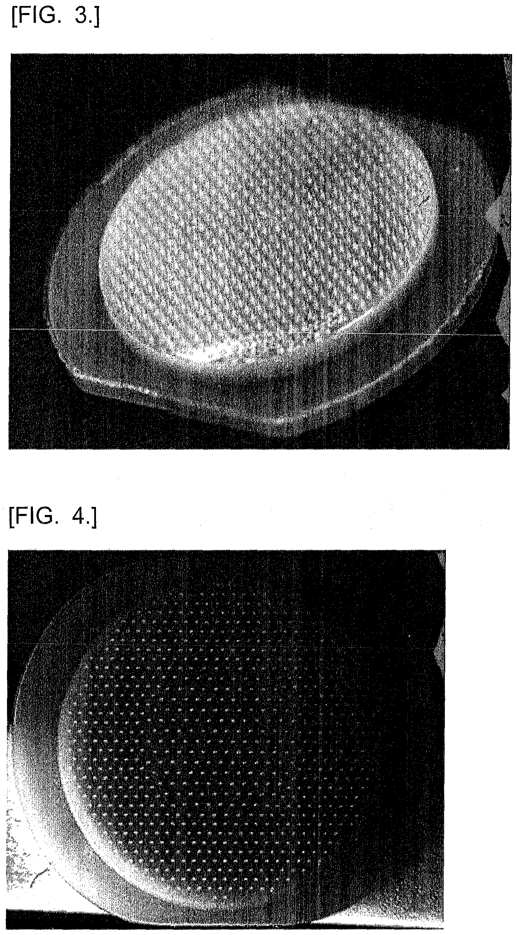

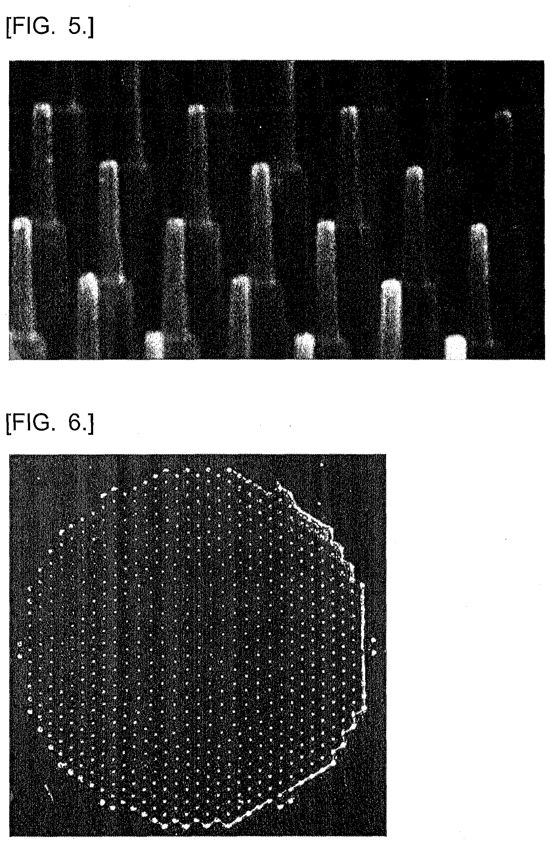

As described above, the microneedles of Patent Documents 1, 2, and 4 to 6 are provided at equal intervals on the substrate, and the needle density per unit area is also constant. On the other hand, Patent Document 3 discloses a needle-shaped body device (microneedle array) including a substrate having a flat face, wherein blades of the needle-shaped body are arranged in a tangential direction of a circle, and an arrangement pattern thereof is a radial shape from a center of the circle toward an outer periphery (FIG. 3), a spiral shape spreading from the center of the circle toward the outer periphery (FIG. 4), a linear shape eccentric from a central axis (FIG. 5(a)), a plurality of non-line-symmetric linear shapes (FIG. 5(b)), and the like. However, blades adjacent to each other in the tangential direction are at equal intervals, and are arranged substantially uniformly in balance over the entire substrate of the needle-shaped body.

Regarding the needle density of the microneedles and the skin permeability of the needles, it is described that a microneedle array having 900 needles/cm² is poor in skin permeability compared with a microneedle array having 400 needles/cm² (Non-Patent Document 2).

### PRIOR ART DOCUMENT

### PATENT DOCUMENT

Patent Document 1: JP 2009-273872 A
Patent Document 2: JP 2010-029634 A
Patent Document 3: JP 2017-074196 A
Patent Document 4: JP 2016-175853 A
Patent Document 5: JP 2015-109963 A
Patent Document 6: JP 2017-047075 A

### NON-PATENT DOCUMENT

Non-Patent Document 1: Ying-shu Quan and Fumio Kamiyama, "The Course of Productization of Microneedle", The Archives of Practical Pharmacy, The Academy of Pharmaceutical Science and Technology, Japan, September 2009, Vol. 69, No. 4, p. 272-276

Non-Patent Document 2: G. Ya, et al., Evaluation needle length and density of microneedle arrays in the pretreatment of skin for transdermal drug delivery, International Journal of Pharmaceutics 391 (2010) 7-12

### SUMMARY OF THE INVENTION

### PROBLEMS TO BE SOLVED BY THE INVENTION

When a conventional microneedle patch is administered to an animal skin for a test, the microneedle patch is struck at a high speed from behind the microneedle patch by an applicator, and energy due to the impact is applied to the microneedle patch to realize microneedle insertion into the skin. The face of the applicator to impact the microneedle patch known to date is a flat face. In the process of administration to an animal or human using such a system, the inventors of the present invention have noticed the following phenomenon.

1. When the density of the needles is too high, insertion into animal and human skin becomes difficult (Phenomenon 1).

According to the findings by the inventors, it tends to be difficult for the needles to penetrate the skin when the needle density is more than 1500 needles/cm². As a matter of course, this number depends on many factors such as the hardness of the skin, the thinness and strength of the needles, and the impact strength of the applicator, and thus is merely a guide. In a microneedle system in which it is important to impregnate (in the case of a dissolvable microneedle) the microneedles with a large amount of valuable material or apply (in the case of an application-type microneedle) a larger amount of valuable material to the microneedles to increase the drug content per unit area, the needle density with which the microneedles can be stably inserted is an extremely important structural factor of the microneedle array.

Prior to the inventors of the present invention, Non-Patent Document 2 describes that a microneedle array having 900 needles/cm² of needles is poor in skin permeability compared with a microneedle array having 400 needles/cm² of needles.

2. In a detailed analysis of Phenomenon 1, it is more difficult to insert the needles into the skin at the central portion of the microneedle array than at the peripheral portion (Phenomenon 2).

A search was conducted on literatures for Phenomenon 2, but no literature pointing out such a phenomenon has been found.

An object of the present invention is to solve a problem that it is difficult for a microneedle located in a central portion of a microneedle array to be inserted into a skin.

### MEANS FOR SOLVING THE PROBLEM

In order to solve the above problem, the inventors of the present invention performed a model experiment related to Phenomenon 2 (difficulty of insertion of the microneedles at the central portion into a skin) using Parafilm instead of a skin. The insertion behavior into Parafilm was examined in detail by stacking eight sheets of Parafilm (thickness: 140 µm), placing a microneedle patch (area = 0.8 cm²) having fine needles with different needle densities on the sheets, and making an impact on the microneedle patch with an applicator (WO 2018/124290 A or JP 2017-185162 A). It is already known in the literature (Int. J. Pharmaceutics 480 (2015) 152-157) to analyze the insertion behavior of microneedles using Parafilm as a skin substitute, and there is a reliable correlation with the insertion behavior into skin.

As a result, the inventors have found that using a microneedle array in which microneedles are arranged such that the needle density is smaller in the central portion than in the peripheral portion allows the total number of needles to increase compared with a microneedle array having a uniform needle density over the entire face, and allows all needles to be stably inserted into a skin, and have completed the present invention. The present invention is as follows.
[1] A microneedle array including a substrate and a plurality of microneedles arranged vertically and horizontally on one face of the substrate, wherein a needle interval of the microneedles is different in a peripheral portion and in a central portion of the substrate, and a needle density of the microneedles is different in the peripheral portion and in the central portion of the substrate.
[2] The microneedle array according to [1], wherein the needle density of the microneedles is sparser in the central portion than in the peripheral portion of the substrate.
[3] The microneedle array according to [1] or [2], wherein the needle interval of the microneedles is wider in the central portion than in the peripheral portion of the substrate.
[4] The microneedle array according to [2], wherein the needle density of the microneedles is 600 to 1500 needles/cm² in the peripheral portion of the substrate and 100 to 800 needles/cm² in the central portion of the substrate, and the needle density is smaller in the central portion of the substrate than in the peripheral portion of the substrate.
[5] The microneedle array according to [2], wherein the needle density of the microneedles is 400 to 2000 needles/cm² in the peripheral portion of the substrate and 0 to 99 needles/cm² in the central portion of the substrate.
[6] The microneedle array according to [2], wherein the needle density of the microneedles is 400 to 2000 needles/cm² in the peripheral portion of the substrate and 0 needles/cm² in the central portion of the substrate, and at least two rows of the microneedles are arranged in the peripheral portion.
[7] The microneedle array according to any one of [1] to [6], wherein the face of the substrate has a circle or ellipse shape, the central portion of the substrate is inside a circumference of 9/10 or less of a radius from a center of the circle or the ellipse, and the peripheral portion of the substrate is outside the central portion.
[8] The microneedle array according to any one of [1] to [6], wherein the face of the substrate has a quadrangle shape, the central portion of the substrate is inside four sides connecting four points at 9/10 or less of diagonal lines from a center of the quadrangle, and the peripheral portion of the substrate is outside the central portion.
[9] A microneedle patch including the microneedle array according to any one of [1] to [8] and an adhesive sheet.
[10] The microneedle patch according to [9], further including a release sheet attached to an adhesive face of the adhesive sheet.

### EFFECT OF THE INVENTION

The microneedle array of the present invention can reliably puncture a skin with a larger number of needles than a microneedle array having a uniform needle density thereby increasing the drug content per unit area and reliably delivering the drug transdermally by setting a density difference in the needle density of the microneedles.

### BRIEF DESCRIPTION OF DRAWINGS

FIG. 1 is a schematic view of a microneedle having a step.
FIG. 2 is a view showing one aspect of a microneedle patch having a release sheet.
FIG. 3 is a photograph of a microneedle array produced in Example 1.
FIG. 4 is an enlarged photograph of the microneedle array produced in Example 1.
FIG. 5 is a micrograph showing the shape of the needles of the microneedle array produced in Example 1.
FIG. 6 is a micrograph of Parafilm after the needles of the microneedle array produced in Example 1 penetrated the Parafilm.
FIG. 7A is an enlarged photograph of a microneedle array produced in Comparative Example 3.
FIG. 7B is an enlarged photograph of the microneedle array produced in Example 2.

### MODES FOR CARRYING OUT THE INVENTION

### Substrate of Microneedle Array

There are no particular limitations on the material, shape, and size of the substrate of the microneedle array, and those conventionally used may be used.

The base of the substrate and the base of the microneedle are basically the same, but different bases may also be used.

Examples of the base include silicon, silicon dioxide, ceramic, glass, metal (stainless steel, titanium, nickel, molybdenum, chromium, cobalt, and the like), and synthetic or natural resin materials. Examples of the synthetic or natural resin material include water-soluble or biodegradable polymers such as polylactic acid, polyglycolic acid, a poly(lactic acid-glycolic acid) copolymer, capronolactone, polyurethane, polyvinylpyrrolidone, hydroxypropyl cellulose, and polyvinyl alcohol, and non-biodegradable polymers such as nylon, polycarbonate, polymethacrylic acid, ethylene vinyl acetate, polytetrafluoroethylene, polyoxymethylene, polyethylene terephthalate, and cyclic olefin polymer (COP). The base may also be a polysaccharide such as hyaluronic acid, sodium hyaluronate, a hyaluronic acid derivative, pullulan, dextran, dextrin, or chondroitin sulfuric acid. One kind of these may be used, or two or more kinds thereof may be used in combination as the base.

The substrate may have any shape. As an example, the shape may be a circle, an ellipse, a triangle, a quadrangle, a polygon, or the like as a basic shape, and may be further modified in accordance with the application site (skin). The size of the substrate is usually 0.2 to 10 cm, preferably 0.5 to 5 cm, when represented by a diameter (long diameter) or a length of one side (long side).

The area of the substrate is usually 0.05 to 100 cm². From the viewpoint of ease of handling, the area is preferably about 0.1 to 10 cm², and more preferably about 0.5 to 5 cm².

The thickness of the substrate depends on the material of the base, and is usually 50 to 2000 µm and preferably 100 to 1000 µm.

### Shape of Microneedle

A microneedle included in the microneedle array has a needle length of 100 µm to 2,000 µm, and preferably 200 to 1,000 µm in order to ensure transdermal absorption of a drug.

When the size of the apex of the tip portion of the needle is represented as a diameter, the diameter is 80 µm or less, preferably 30 µm or less, in order to facilitate insertion into the skin and reduce the drug residue on the skin.

Each microneedle may have a cylinder or circular cone shape with a circular bottom face, an elliptic cylinder or elliptic cone shape with an elliptic bottom face, a triangular prism or triangular pyramid shape with a triangle bottom face, a quadrangular prism or quadrangular pyramid shape with a quadrangular bottom face, and a polygonal prism or polygonal pyramid shape with a polygonal bottom face. In the case of an ellipse, the size of the bottom face is represented by a major axis as a diameter, and the minor axis is shorter than the major axis as long as an ellipse can be formed. In the case of a triangle or a polygon, one side may be represented as the size, or a diagonal line may be represented as the size. When the microneedle has a circular cone shape, the diameter at the bottom face is about 100 to 400 µm and preferably about 150 to 300 µm.

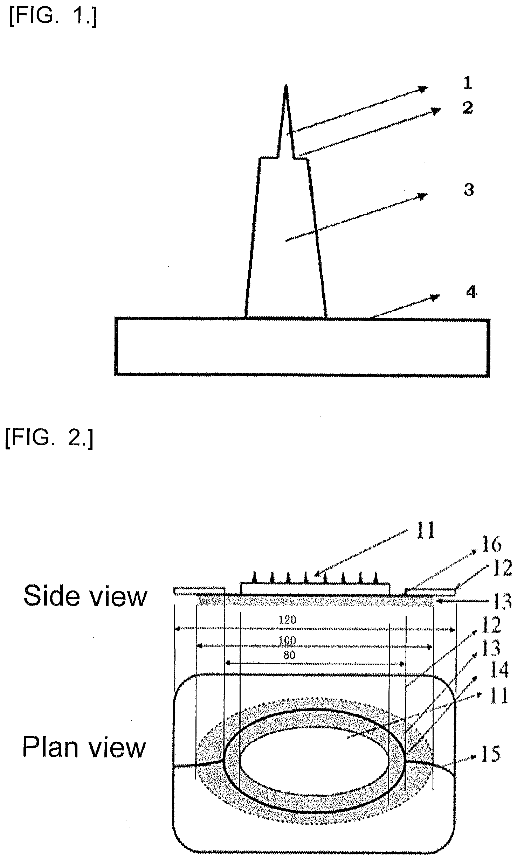

The microneedle in the present invention may have a step. Here, the step refers to a portion where a cross-sectional area of the microneedle is reduced discontinuously from a certain point of the microneedle toward the tip and where a cross section has a stepwise shape as shown in FIG. 1. The shape of the microneedle having a step will be described with reference to FIG. 1. In a stepped microneedle, it is preferable that the length of a tip portion 1 is 50 to 500 µm and the rest is defined as a bottom portion 3. The size of a margin 2 of the step between the tip portion and the bottom portion is preferably larger than 10 µm and smaller than 100 µm. The size is more preferably 14 to 50 µm. A substrate of the microneedle array is numbered 4.

The margin 2 of the step is a face perpendicular to the axis of the microneedle (face parallel to the substrate) within the range of machining accuracy. The size of the margin 2 of the step refers to a difference in radius between the tip portion and the bottom portion of the step. The tip portion and the bottom portion differ depending on the shape of the microneedle.

As a preferred aspect, the microneedle of the present invention has a circular cone shape. The total length of the microneedle (needle length) is preferably about 70 to 1000 µm. In the stepped microneedle, it is preferable that the length of the tip portion 1 is 50 to 500 µm and the rest is defined as the bottom portion (two-step needle), or the rest is defined as an intermediate portion and the bottom portion in a three-step needle. The size of the margin 2 of the step between the tip portion and the intermediate portion and between the intermediate portion and the bottom portion is preferably larger than 10 µm and smaller than 100 µm. The size is more preferably 14 to 50 µm.

### Arrangement of Microneedles on Substrate

The microneedle array of the present invention includes a plurality of microneedles arranged vertically and horizontally on one face of a substrate, wherein a needle interval of the microneedles is different in a peripheral portion and in a central portion of the substrate, and a needle density of the microneedles is different in the peripheral portion and in the central portion of the substrate.

It is preferable that the needle density of the microneedles is sparser in the central portion than in the peripheral portion of the substrate from the viewpoint of reliable skin puncture with the needles. The needle interval of the microneedles is also preferably wider in the central portion than in the peripheral portion of the substrate.

As a suitable example, the needle density of the microneedles is 600 to 1500 needles/cm² in the peripheral portion of the substrate and 100 to 800 needles/cm² in the central portion of the substrate. More preferably, the density is 700 to 1000 needles/cm² in the peripheral portion of the substrate, and is 300 to 800 needles/cm² in the central portion of the substrate. Here, a numerical value smaller than the needle density in the peripheral portion of the substrate is selected as the needle density in the central portion of the substrate.

As another suitable example, the needle density in the central portion of the substrate may be less than 100 needles/cm², specifically 0 to 99 needles/cm². In this case, the density is 400 to 2000 needles/cm² and more preferably 600 to 1500 needles/cm² in the peripheral portion of the substrate.

The needle interval of the microneedles may be appropriately set from the ratio between the area of the central portion and the area of the peripheral portion of the substrate and the needle density of the microneedles.

When the face of the substrate is a circle or an ellipse, it is preferable that the central portion of the substrate is inside a circumference of 9/10 or 2/3 or less of the radius from the center of the circle or the ellipse, and the peripheral portion of the substrate is outside the central portion based on the observation result of the region where the skin insertion of the microneedle array is difficult.

When the face of the substrate is a quadrangle, it is preferable that the central portion of the substrate is inside four sides connecting four points at 9/10 or 2/3 or less of the diagonal lines from the center of the quadrangle, and the peripheral portion of the substrate is outside the central portion.

It is preferable that at least two rows of microneedles are arranged in the peripheral portion.

### Microneedle Patch

The microneedle patch of the present invention includes the microneedle array and an adhesive sheet. The adhesive sheet is typically obtained by using polyurethane, polyethylene, polyester, paper, or the like as a base material of a film, and applying an acrylic or rubber-based adhesive of about 5 to 50 µm onto the film molded to have a thickness of about 5 to 50 µm. The shape of the adhesive sheet is not particularly limited, and is preferably a circle, an ellipse, a bead, or the like similar to the shape of the microneedle array.

The microneedle patch of the present invention may further include a release sheet attached to an adhesive face of the adhesive sheet to protect the adhesive face of the adhesive sheet and hold a soft microneedle array for easy handling. As the release sheet, a protective release sheet disclosed in JP 2014-028108 A may be used. A specific example of the microneedle patch having a release sheet is shown in FIG. 2.

### Drug Held by Microneedles

The microneedle array of the present invention may contain a drug in the base when the base of the microneedle is a water soluble polymer. Alternatively, the microneedle array of the present invention may have a drug coat layer at the tip portion of the microneedle.

Here, the drug includes all compounds that work on a skin or penetrate a skin and produce some beneficial action. Examples of drugs suitable for the purpose of the present invention include physiologically active peptides and derivatives thereof, nucleic acids, oligonucleotides, various antigenic proteins, bacteria, and viral fragments. Examples of the physiologically active peptides and derivatives thereof include calcitonin, adrenocorticotropic hormone, parathyroid hormone (PTH), hPTH (1 → 34), insulin, exendin, secretin, oxytocin, angiotensin, β-endorphin, glucagon, vasopressin, somatostatin, gastrin, luteinizing hormone releasing hormone, enkephalin, neurotensin, atrial natriuretic peptide, growth hormone, growth hormone releasing hormone, bradykinin, substance P, dynorphin, thyroid-stimulating hormone, prolactin, interferon, interleukin, G-CSF, glutathione peroxidase, superoxide dismutase, desmopressin, somatomedin, endothelin, and salts thereof. Examples of the antigenic proteins include influenza antigens, HBs surface antigens, HBe antigens, and the like.

The drug may be a cosmetic product.

In the case of a microneedle array having a drug coat layer at the tip portion of a microneedle, the lower end of the drug coat layer may be 50 µm or more from the root of the needle, and the upper end may have any height depending on the application amount of the drug. Preferably, the upper end is at the tip of the microneedle, but the drug does not necessarily have to be applied up to the end of the tip. The length of the drug coat layer is typically 50 µm to 800 µm, and preferably 150 µm to 600 µm.

The lower end and the upper end of the drug coat layer are values obtained by measuring the lower end and the upper end of the microneedle to which the drug is applied in the vertical direction from the substrate of the microneedle array, respectively. The length of the drug coat layer is represented by the difference between the lower end and the upper end of the microneedle to which the drug is applied.

On the other hand, the thickness of the drug coat layer varies depending on the drug application liquid and the number of times of application.

When the tip of the microneedle is immersed in a drug aqueous solution to apply a drug to the tip of the microneedle, it is desirable that a base material substance is dissolved in the drug aqueous solution, and the drug is held by the microneedle together with the base material substance when the drug is dried after application. The base material substance is required to be a substance that does not impair the stability of the drug, and examples thereof include: polymer substances, for example polymeric polysaccharides such as hyaluronic acid, dextrin, dextran, chondroitin sulfate sodium, hydroxypropyl cellulose, ethyl cellulose, and carboxymethyl cellulose Na salt, proteins such as collagen, water-soluble synthetic polymers such as polyvinyl pyrrolidone and polyvinyl alcohol; low molecular weight saccharides such as glucose, sucrose, maltose, and trehalose; and mixtures thereof. A drug aqueous solution to which the base material substance and a water-soluble salt are added is suitable.

Here, the water-soluble salt is preferably a water-soluble salt such as sodium chloride or zinc chloride.

The concentration of the base material substance in the drug aqueous solution is desirably 2 mass% to 60 mass%. When the concentration is lower than 2 mass%, the viscosity of the drug aqueous solution is small, and the application adhesion amount at the time of immersion is small. When the concentration is 60 mass% or more, the concentration of the drug aqueous solution is too high, and drug application is not stable. The ratio of the polymer and the low molecular weight saccharide in the base material may be changed depending on the properties of the drug. When the drug is a polymer drug, all of the base may be a low molecular weight saccharide.

An antioxidant, a surfactant, or the like may be added to the aqueous drug solution as necessary. In addition, glycerin, ethylene glycol, and a low molecular weight polymer thereof may be added to further enhance dissolution of the drug in the skin.

### Method for Producing Microneedle Array

### (1) Processing of Die

The die used for producing the microneedle array of the present invention may be produced by wet etching processing or dry etching processing using a silicon substrate, precision machining (electrical discharge machining, laser machining, hot embossing, injection molding, etc.) using metal or resin, machine cutting processing, or the like.

### (2) Step of Molding Microneedle Array

A microneedle array made of a water-soluble polymer may be mass-produced using a mold (die). Examples thereof include a method including casting an aqueous solution containing a water-soluble polymer, and a drug and other components as necessary, drying the aqueous solution, thereafter peeling off the resulting product (JP 2009-273872 A [0029] to [0031]).

A microneedle made of a polymer that can be injection-molded may be produced by injection-molding a material using a die (JP 2003-238347 A [0017] and [0018]). As the injection molding die, stainless steel, heat-resistant steel, superalloy, or the like may be used. The die has recesses corresponding to 100 to 1500 of microneedles per square centimeter to form the shape of the microneedles. To form the recesses, micromachining means such as laser or electrical discharge machining may be used.

As one aspect of a method for producing a microneedle array made of a polymer that can be injection-molded (for example, thermoplastic resin), a method including supplying pellets made of a thermoplastic resin material to an injection molding machine equipped with a microneedle injection molding die, and injection-molding a microneedle array at a cylinder temperature of 230 to 280°C, a die temperature of 60 to 130°C, and an injection pressure of 1000 to 1500 KPa may be an example.

As the thermoplastic resin material, polyglycolic acid, polylactic acid, or a copolymer thereof may be used singly or as a mixture. Furthermore, a composition containing an inorganic filler, another thermoplastic resin, and the like may be used as long as the object of the present invention is not impaired.

As a preferred specific example, a composition (compound) obtained by blending 0 to 20 parts by mass of an inorganic filler, 0 to 30 parts by mass of another thermoplastic resin, and the like with respect to 100 parts by mass of polyglycolic acid may be used. If the amount of the inorganic filler or the other thermoplastic resin is more than 20 parts by mass, the resulting injection molded product may be insufficient in impact strength and toughness, and melt processability may deteriorate.

Examples of the inorganic filler include silica, titanium oxide, calcium carbonate, and calcium silicate. These may be used singly or in combination of two or more kinds thereof.

Examples of the other thermoplastic resin include homopolymers and copolymers of ε-caprolactone and TPX. These thermoplastic resins may be used singly or in combination of two or more kinds thereof. The other thermoplastic resin is usually used at a proportion of 0 to 30 parts by mass with respect to 100 parts by mass of polyglycolic acid, for example.

The microneedle array obtained by injection molding is taken out from the die after cooling.

### EXAMPLES

Examples of the present invention are shown below. The present invention is not limited to Examples.

### Example 1

A die was attached to an injection molding machine (FANAC CORPORATION), and polyglycolic acid was melted to perform injection molding. The injection molding was performed at a cylinder temperature of 235°C, an injection pressure of 1350 kPa, and a die temperature of 120°C, and a milky white microneedle array having a diameter of 10 mm was taken out (FIG. 3). An enlarged view is shown in FIG. 4.

The needle density was different in the central portion and in the peripheral portion of the substrate. The circular portion having a diameter of about 5 mm in the central portion had a needle density of 750 needles/cm², and had a total of 169 two-step microneedles. The peripheral portion (a portion from 5 mm from the center to an outer diameter (10 mm)) had a needle density of 960 needles/cm², and had a total of 576 two-step microneedles. A micrograph showing the shape of the needles is shown in FIG. 5. Each needle was a two-step needle having a tip portion length of 30 µm.

The microneedle array was attached to a spring-loaded applicator. The spring constant of the applicator was 0.516 N/mm². Five sheets of Parafilm (manufactured by LMS, thickness: 170 µm) were stacked on a silicon plate having a thickness of 1 cm to form a skin model, and the microneedle array was impact-administered thereon with the applicator. The microneedle array was peeled off from the sheets of Parafilm, and the penetration state of the needles at the second sheet from the surface was observed with a microscope. In the case where the needles penetrate the second sheet, it is predicted that the needles reach a depth of approximately 350 µm from the skin. The result is shown in FIG. 6. From FIG. 6, it was found that all the needles had penetrated the second sheet.

### Comparative Examples 1 and 2

A microneedle array was produced in the same manner as in Example 1 except that a die different from that in Example 1 (in Comparative Example 1, the needle density is uniform at 750 needles/cm², and in Comparative Example 2, the needle density is uniform at 960 needles/cm²) was used in Example 1. With the obtained microneedle arrays, a parafilm penetration test was performed in the same manner as in Example 1. The results of Example 1 and Comparative Examples 1 and 2 are shown in Table 1.

**[Table 1]**

| | Needle density in central portion (needles/cm²) | Needle density in peripheral portion (needles/cm²) | Total number (needles) | Parafilm penetrability |
|---|---|---|---|---|
| Ex. 1 | 750 | 9 6 0 | 745 | ○ |
| Comp.Ex.1 | 750 | 750 | 675 | ○ |
| Comp.Ex.2 | 960 | 960 | 760 | × |

○: All needles penetrated two sheets of Parafilm.
×: Some needles did not penetrate two sheets of Parafilm.

Comparative Examples 1 and 2 are microneedle arrays having no density difference. Each of the arrays had a diameter of 10 mm. By setting the density difference in the needle density of the microneedle as in Example 1, a result was obtained from which a prediction that more needles can reliably puncture the skin was made.

### Examples 2, 3, and 4 and Comparative Examples 3 and 4

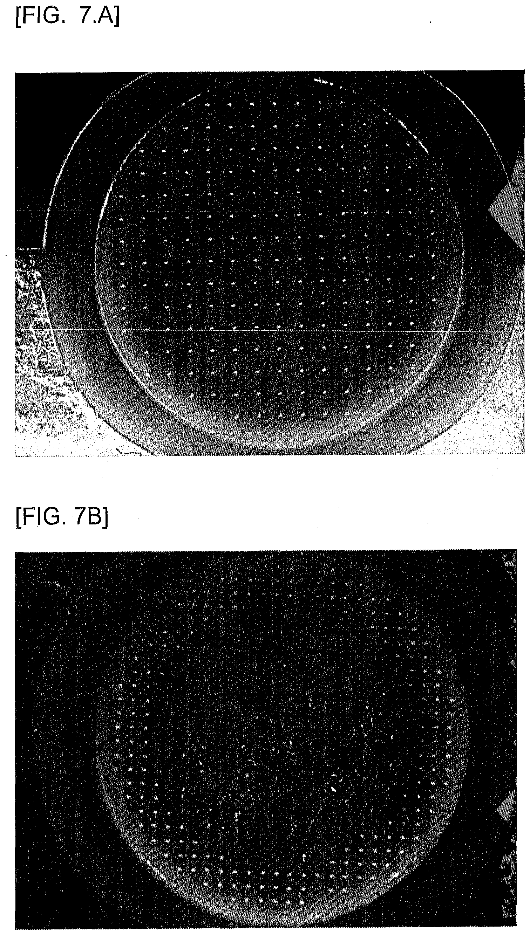

A die was attached to an injection molding machine (FANAC CORPORATION), and polyglycolic acid was melted to perform injection molding. The injection molding was performed at a cylinder temperature of 235°C, an injection pressure of 1350 kPa, and a die temperature of 120°C to produce 4 types of milky white microneedle arrays each having a diameter of 10 mm. The total length of the needle was all 600 µm. The enlarged micrographs are shown in FIGS. 7A and 7B.

No. 1 is a microneedle array having a needle interval of 650 µm and an entirely uniform needle arrangement (Comparative Example 3, FIG. 7A). No. 2 is a microneedle array in which the needle interval is 400 µm and the needle arrangement is concentrated in the outer periphery (Example 2, FIG. 7B). The number of needles was unified to 193 in No. 1 and No. 2.

No. 3 is a microneedle array having a needle interval of 400 µm and an entirely uniform needle arrangement (Comparative Example 4). No. 4 is a microneedle array in which the needle interval is 350 µm and the needle arrangement is concentrated in the outer periphery (Example 3). The number of needles was unified to 489 in No. 3 and No. 4.

No. 5 is a microneedle array in which the needle interval is 400 µm and the needle arrangement is concentrated in two rows in the outer periphery (Example 4). The number of needles was 110. No. 5 was produced and evaluated in consideration of the need for a microneedle array with a small number of needles depending on the application.

The microneedle array was attached to a spring-loaded applicator. The spring constant of the applicator was 0.516 N/mm². Five sheets of Parafilm (manufactured by LMS, thickness: 130 µm) were stacked on a silicon plate having a thickness of 1 cm to form a skin model, and the microneedle array was impact-administered thereon with the applicator. The microneedle array was peeled off from the sheets of Parafilm, and the penetration state of the needles at the first to fourth sheet from the surface was observed with a microscope. In the case where the needles penetrate the third sheet, it is predicted that the needles reach a depth of approximately 390 µm from the skin. The results are shown in Table 2.

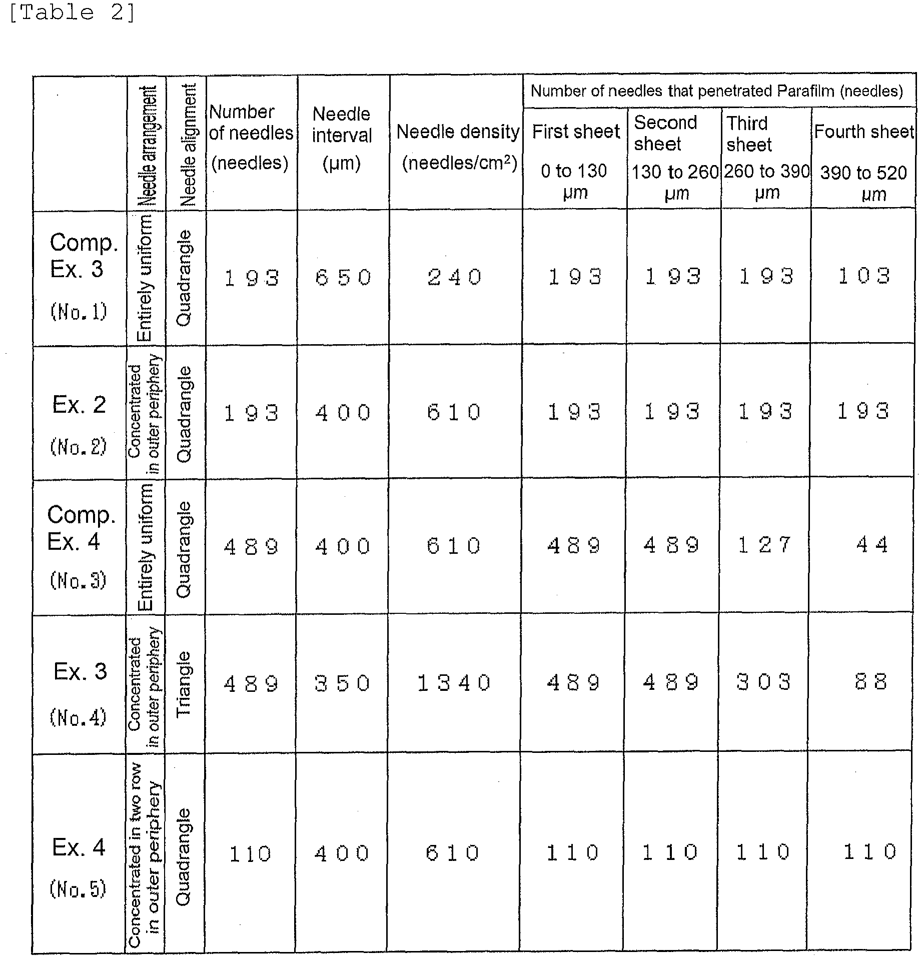

In Example 2 and Comparative Example 3, the number of needles of the microneedle array is the same. In Comparative Example 3 in which the needle arrangement was uniform, all the needles penetrated up to the third sheet of Parafilm, but in Example 2 in which the needle arrangement was concentrated in the outer periphery, all the needles penetrated up to the fourth sheet of Parafilm. By setting the needle density in the central portion to 0, a result was obtained from which a prediction that skin puncture was reliably performed was made.

Similarly, in Example 3 and Comparative Example 4, the number of needles of the microneedle array is the same, but the needle density is higher than that in Example 2 and Comparative Example 3. In Comparative Example 4 in which the needle arrangement was uniform, the number of needles that penetrated the third sheet of Parafilm decreased to 1/4 of the total, whereas in Example 3 in which the needle arrangement was concentrated in the outer periphery, the number of needles that penetrated the third sheet of Parafilm was about 3/4 of the total. By concentrating the needle arrangement in the outer periphery even in the microneedle array having a high needle density, a result was obtained from which a prediction that skin puncture was reliably performed was made.

In Example 4, the needle interval and the needle density were the same as those in Example 2, but the number of needles was decreased and the needles were concentrated in two rows on the outer periphery. As in Example 2, all the needles penetrated up to the fourth sheet of Parafilm. By setting the needle density in the central portion to 0, a result was obtained from which a prediction that skin puncture was reliably performed was made.

### DESCRIPTION OF REFERENCE SYMBOLS

- 1: Tip portion
- 2: Margin of step
- 3: Bottom portion
- 4: Substrate of microneedle array
- 11: Microneedle array
- 12: Release sheet
- 13: Adhesive sheet
- 14: Hole
- 15: Cutting line
- 16: Gap

## Claims

1. A microneedle array comprising a substrate and a plurality of microneedles arranged vertically and horizontally on one face of the substrate,
wherein a needle interval of the microneedles is different in a peripheral portion and in a central portion of the substrate, and a needle density of the microneedles is different in the peripheral portion and in the central portion of the substrate.

2. The microneedle array according to claim 1, wherein the needle density of the microneedles is sparser in the central portion than in the peripheral portion of the substrate.

3. The microneedle array according to claim 1 or 2, wherein the needle interval of the microneedles is wider in the central portion than in the peripheral portion of the substrate.

4. The microneedle array according to claim 2, wherein the needle density of the microneedles is 600 to 1500 needles/cm² in the peripheral portion of the substrate and 100 to 800 needles/cm² in the central portion of the substrate, and the needle density is smaller in the central portion of the substrate than in the peripheral portion of the substrate.

5. The microneedle array according to claim 2, wherein the needle density of the microneedles is 400 to 2000 needles/cm² in the peripheral portion of the substrate and 0 to 99 needles/cm² in the central portion of the substrate.

6. The microneedle array according to claim 2, wherein the needle density of the microneedles is 400 to 2000 needles/cm² in the peripheral portion of the substrate and 0 needles/cm² in the central portion of the substrate, and at least two rows of the microneedles are arranged in the peripheral portion.

7. The microneedle array according to any one of claims 1 to 6, wherein the face of the substrate has a circle or ellipse shape, the central portion of the substrate is inside a circumference of 9/10 or less of a radius from a center of the circle or the ellipse, and the peripheral portion of the substrate is outside the central portion.

8. The microneedle array according to any one of claims 1 to 6, wherein the face of the substrate has a quadrangle shape, the central portion of the substrate is inside four sides connecting four points at 9/10 or less of diagonal lines from a center of the quadrangle, and the peripheral portion of the substrate is outside the central portion.

9. A microneedle patch comprising the microneedle array according to any one of claims 1 to 8 and an adhesive sheet.

10. The microneedle patch according to claim 9, further comprising a release sheet attached to an adhesive face of the adhesive sheet.
